# EUROPEAN PATENT APPLICATION

(11) **EP 4 477 637 A1**
(43) Date of publication of application: **18.12.2024**
(21) Application number: 24182439.0
(22) Date of filing: 14.06.2024
(51) Int. Cl.: C05G 3/90

(54) **METHOD FOR SLOWING DOWN DECOMPOSITION OF UREA CONTAINED IN NITROGEN FERTILISERS AND NITROGEN FERTILISER WITH UREASE INHIBITOR SLOWING DOWN UREA DECOMPOSITION**

(30) Priority: 15.06.2023 PL 44523823
(71) Applicant: "Nano-Tech Polska" Spolka z ograniczona odpowiedzialnoscia Spolka komandytowa, 04-987 Warszawa (PL)
(72) Inventor: MALEK, Jaroslaw, 00-891 Warszawa (PL); SIEJKO, Pawel, 05-410 Jozefow (PL); TUL, Andrzej, 03-349 Warszawa (PL); PAWLOWSKA, Marta, 05-500 Piaseczno (PL); RUTKOWSKA, Beata, 02-495 Warszawa (PL); SZULC, Wieslaw, 02-776 WArszawa (PL)
(74) Representative: Kacperski, Andrzej

(57) **Abstract**

Method for slowing down the decomposition of urea in the soil contained in nitrogen fertilisers used for affecting plants, by applying a urease inhibitor - an enzyme that catalyses the decomposition of urea in the fertiliser to ammonia and carbon dioxide during the hydrolysis process occurring in the soil, in which the urease inhibitor used is non-ionic silver particles, obtained by a physical method, without impurities and with a purity above 99.99%, in the form of particles with an expanded active surface area, various sizes, and a number distribution of these sizes from 1 nm to 500 nm, wherein the silver particles are dosed into the fertiliser mass in the form of a colloid containing the mentioned silver particles in deionized water, to achieve a concentration in the fertiliser mass from 1 to 50 ppm.

## Description

The subject of the invention is a method for slowing down decomposition of urea contained in nitrogen fertilisers used for affecting plants in the soil and a nitrogen fertiliser with a urease inhibitor that slows the decomposition of urea contained in it. Nitrogen fertilisers according to the invention are intended for use particularly for cereal crops, including wheat, corn, and rapeseed.

Nitrogen is the most important nutrient in plant nutrition, determining the size and quality of the yield. This element is the most yield-forming nutrient, influencing both biomass and seed yield.

Nitrogen supplied to the soil with fertilisers is partially taken up by plants, partially by microorganisms present in the soil, and some of this element is leached by rainwater. Nitrogen losses due to leaching mainly depend on the form of this element in the fertiliser - nitrate nitrogen is the easiest to leach. Urea is a universal nitrogen fertiliser, containing nitrogen in the amide form. In the soil, urea converts to the ammonium form and then to the nitrate form. From the perspective of plant nitrogen uptake, it is important to match it to the plant's nutritional requirements.

After applying urea fertiliser to the soil, due to moisture and the urease enzyme, urea hydrolysis occurs almost immediately, releasing ammonia into the environment. This phenomenon is unfavourable from an environmental protection perspective, as urea decomposes in a short time, releasing large amounts of ammonia, followed by the formation of nitrites and nitrates, which, if not utilized by plants, pose a threat to the environment and water. This results in greater use of nitrogen fertilisers in agriculture, which also has economic consequences.

For this reason, substances that inhibit the enzymatic breakdown of urea are added to urea fertilisers. By using a urease inhibitor, the process of ammonia release is slower, and plants gradually take up nitrogen compounds necessary for proper growth.

Slowed hydrolysis of urea fertilisers ensures that plants have a steady supply of nitrogen and absorb it in the quantities they need, preventing over-fertilization and mitigating the risk of nitrate leaching into the soil.

According to [Klimczyk, M., Siczek, A., & Schimmelpfennig, L. (2021). Improving the efficiency of urea-based fertilization leading to reduction in ammonia emission. Science of the Total Environment, 771, 145483], urease inhibitors delay urea hydrolysis in the soil and thus limit the increase in soil pH and ammonia/ammonium ion concentration near the fertiliser granules, thereby reducing the toxic effect of high ammonia concentration on seed germination. The mechanism of urea decomposition occurs in two stages. In the first stage, catalysed by the urease enzyme, a complex compound containing carbamic acid and ammonia is formed. In the second stage, this complex spontaneously decomposes to ammonia and carbonic acid. Examples of compounds that inhibit the hydrolysis process are: NBPT (N-(n-butyl)thiophosphoric triamide), NPPT (N-(n-propyl)thiophosphoric triamide), and ATS (ammonium thiosulfate). NBPT and NPPT are considered significantly more effective than ATS, as they delay the hydrolysis process and consequently the peak ammonia emission by about a week.

NBPT is regarded as the most effective among many inhibitors that slow down the microbiological decomposition of ammonia. Its mechanism of action involves blocking the active centre of urease by forming a bond with nickel and oxygen atoms, reducing the likelihood of urea binding with the nickel atom. After applying NBPT, the fertiliser's effect is prolonged for a period of 2 to 10 weeks.

Urea-based fertilisers containing the urease inhibitor N-(n-butyl)thiophosphoric triamide (NBPT), such as UREAstabil and moNolith46^{®}, are available on the market. Liquid preparations with a urease inhibitor, such as Agrotain^{®}, containing two active ingredients: NBPT (25%) and N-methylpyrrolidone (10%), are also available. Another example is Limus^{®} Clear, which combines the effects of two active substances, NBPT and NPPT (N-(n-propyl)thiophosphoric acid triamide), reducing nitrogen losses from urea-based fertilisers.

For instance, urease inhibitors based on thiophosphoric triamide compounds are disclosed in patent US 4,530,714, which includes N-(n-butyl)thiophosphoric acid triamide (NBPT).

In turn, patent EP 2 885 261 presents improved solvent systems for producing liquid formulations of urease inhibitors or nitrification inhibitors, particularly NBPT, containing alkylene glycol alkyl ethers. Specifically, it presents a composition in which the urease inhibitor is a phosphoric or thiophosphoric triamide, and the alkylene glycol alkyl ether solvent is selected from the group consisting of diethylene glycol monobutyl ether, diethylene glycol monomethyl ether, dipropylene glycol dimethyl ether, triethylene glycol monobutyl ether, tripropylene glycol monomethyl ether, and tetraethylene glycol monobutyl ether.

Standard urease inhibitors must be used correctly and at the right time to be effective. Their improper use or timing can reduce their effectiveness or negate the benefits of their use, which is a certain limitation in their application. Standard urease inhibitors do not always provide consistent benefits under all soil and environmental conditions. Their effectiveness can vary depending on factors such as soil pH, temperature, moisture, and the specific urease inhibitor used.

All these factors justify the search for new means of stimulating plant growth, directing interest toward new materials in the form of metal particles and new ways of applying them.

[Mehta, C. M., Srivastava, R., Arora, S., & Sharma, A. K. (2016). Impact assessment of silver nanoparticles on plant growth and soil bacterial diversity. 3 Biotech, 6, 1-10] demonstrated that foliar application of metal particles positively affects plant growth. The most favourable growth conditions for cowpea (*Vigna sinensis,* var. Pusa Koma) were noted after soil application of particles at a level of 50 ppm. The application of particles positively affected root growth, plant mass, and an increase in the number of root nodules was also noted. For brassicas (*Brassicajuncea,* var. Pusa Jai Kisan), improved shoot parameters were noted at a dose of 75 ppm (diameter, length, mass), while it did not significantly affect root parameters.

In contrast, [Sillen, W. M., Thijs, S., Abbamondi, G. R., Janssen, J., Weyens, N., White, J. C., & Vangronsveld, J. (2015). Effects of silver nanoparticles on soil microorganisms and maize biomass are linked in the rhizosphere. Soil Biology and Biochemistry, 91, 14-22.] found that after applying silver particles to the soil, maize biomass increased significantly. Increases in leaf length, root biomass, and shoot biomass were noted.

Meanwhile, [Sharma, P., Bhatt, D., Zaidi, M. G. H., Saradhi, P. P., Khanna, P. K., & Arora, S. (2012). Silver nanoparticle-mediated enhancement in growth and antioxidant status of Brassica juncea. Applied Biochemistry and Biotechnology, 167, 2225-2233] noted the positive effects of silver particles on the growth and antioxidant status of 7-day-old *Brassica juncea* seedlings. Compared to the control, the application of particles resulted in increased root length and seedling vigor index by 326% and 133%, respectively. There was also an increase in fresh biomass and shoots, as well as improved quantum efficiency of photosynthesis and higher chlorophyll content compared to the control seedlings. Additionally, it was noted that the levels of malondialdehyde and hydrogen peroxide decreased in the treated seedlings. It was also shown that the particles induced the activity of specific antioxidant enzymes, resulting in a reduction in reactive oxygen species levels. The decrease in proline content confirmed the improvement in the antioxidant status of the treated seedlings. It was found that the observed stimulating effect of silver particles is dose-dependent, with 50 ppm being the most favourable dose for plant growth stimulation.

Moreover, [Iqbal, M., Raja, N. I., Mashwani, Z. U. R., Hussain, M., Ejaz, M., & Yasmeen, F. (2019). Effect of silver nanoparticles on growth of wheat under heat stress. Iranian Journal of Science and Technology, Transactions A: Science, 43, 387-395] demonstrated that the use of silver particles can protect wheat from heat stress by improving morphological growth. Although silver particles increased morphological growth in all tested combinations (25, 50, 75, and 100 ppm), statistically significant results were observed at 50 and 75 ppm. In the studies by [Salama, H. M. (2012). Effects of silver nanoparticles in some crop plants, common bean (Phaseolus vulgaris L.) and corn (Zea mays L.). Int Res J Biotechnol, 3(10), 190-197] an increase in shoot and root length was noted with increasing particle concentration. The best results were obtained for a dose level of 60 ppm. For the given concentration, compared to the control, there was an increase in shoot and root length of 19 and 21% for beans (*Phaseolus vulgaris* L,) respectively, while for maize (Zea *mays* L.) these values were 12 and 18%. Fresh and dry biomass, on the other hand, increased by 30 and 27% and 35 and 33% for beans and maize, respectively, compared to the control sample. Increases in leaf area, chlorophyll, carbohydrate and protein content were recorded for both test crops for doses up to 60 ppm. Above the recommended dose, a marked decrease was noted for all indicators.

From patent EP 3 302 063, a method of stimulating seeds of dicotyledonous plants is known in which the seeds are soaked in a solution containing metal nanoparticles and then dried, characterised in that the seeds are soaked in a nanocolloidal metal solution containing non-ionic metal nanoparticles selected from: silver (Ag), gold (Au), copper (Cu) and platinum (Pt), obtained by a physical method, at concentrations ranging from 0.05 ppm to 50 ppm, to achieve 40-60 wt.%. water content, and then dried at room temperature to obtain 10-40 wt% water content. A non-ionic nanocolloidal solution of metal in deionised water shall be used. The seeds of the following plants are used as seeds of dicotyledonous plants: winter oilseeds resistant to cold, sown in early spring and sensitive to a limited degree to cold, long-germinating vegetable seeds, in particular seeds of: rape, sugar beet, pepper, carrot, parsley, celery.

The aim of the invention is to use metals to stimulate plant growth through the application of metal particles in nitrogen fertilisers in the soil, eliminating the need for foliar feeding, while minimising the amount of active substance affecting plant growth. Supplementation should take place as a process spread over time, with limited aggressive environmental impact.

The solution is to use the method and product to which the invention relates. Supplementation according to the invention is carried out in the soil. It takes advantage of slowing down the decomposition of urea contained in the nitrogenous fertilisers used to affect plants, by using, in the urea hydrolysis process taking place in the soil, an inhibitor of urease - an enzyme catalysing the reaction of decomposition of urea contained in the fertiliser to ammonia and carbon dioxide.

The essence of the method according to the invention is that non-ionic silver particles, obtained by a physical method, without impurities and with a purity of more than 99.99 %, in the form of particles with a developed active surface, different sizes and a number distribution of these sizes from 1 nm to 500 nm, are used as a urease inhibitor, whereby the silver particles are dosed into the fertiliser mass in the form of a colloid containing the said silver particles in deionised water, until a concentration in the fertiliser mass of 1 to 50 ppm is obtained. Nitrogen fertiliser treatment is carried out on spring-sown crops and can be used for cereal crops, including wheat, maize and oilseed rape.

The essence of the urea fertiliser according to the invention is that the agglomerated fertiliser contains, as a urease inhibitor, non-ionic silver particles, obtained by a physical method, with a purity of more than 99.99 %, in the form of particles with a developed active surface and a number size distribution from 1 nm to 500 nm, initially introduced in the form of a colloid containing said silver particles in deionised water, with a concentration in the mass of the fertiliser from 1 ppm to 50 ppm.

The essence of the invention is also the use of non-ionic silver particles, obtained by a physical method, without impurities and with a purity above 99.99%, in the form of particles with an expanded active surface area, various sizes, and a numerical distribution of these sizes from 1 nm to 500 nm, introduced into the fertiliser in the form of a colloid containing the mentioned silver particles in deionized water, with a concentration in the fertiliser mass from 1 ppm to 50 ppm, as a urease inhibitor that slows the decomposition of urea contained in the nitrogen fertiliser - to slow down and extend the gradual uptake of nitrogen by plants.

Although the effects of silver, including its particles, on plants are known, it was unexpectedly found that only the use of non-ionic particles obtained by physical means and without admixtures - with a purity of at least 99.99 % and also in a form with a developed active surface - leads to the possibility of using such silver particles as a urease inhibitor. Thus, it is possible to eliminate urease inhibitors in the form of chemical compounds. In addition, it is possible to achieve a synergistic effect of such silver particles as a plant growth stimulator.

In addition to the urease inhibition itself, the described method and product according to the invention exhibit further properties described below with the test results.

The dose of colloidal particles proposed according to the invention is considerably lower than that commonly used for other urease inhibitors. For NBPT, for example, the active ingredient content ranges from 0.09 to 0.2% (w/w) and for NPT it is in the range of 0.04 to 0.15% (w/w). In contrast, in the case of ammonium thiosulphate (ATS, (NH₄)₂S₂O₃), currently used for nitrogen and sulphur supplementation of soils, the doses used for urease inhibition are much higher and range from 2500 to 5000 mg/kg soil.

In addition, according to the data in [Byrne, M. P., Tobin, J. T., Forrestal, P. J., Danaher, M., Nkwonta, C. G., Richards, K., ... & O'Callaghan, T. F. (2020). Urease and nitrification inhibitors-As mitigation tools for greenhouse gas emissions in sustainable dairy systems: a review. Sustainability, 12(15)], 6018, the half-life (DT50) for NBPT (the most commonly used urease inhibitor) and thus the inhibition period of the enzyme is strongly dependent on the pH of the matrix. At pH 3 it is 58 minutes, for pH=7 it is 92 d and for pH=1 1 it is equal to 16 d.

At the same time, the described method and the product according to the invention, when using the indicated form, amount and concentration of non-ionic silver particles, does not disturb the biological balance in the ecosphere.

The method presented according to the invention for slowing down the decomposition in the soil of urea contained in nitrogen fertiliser and the urea fertiliser presented according to the invention with a urease inhibitor for slowing down the decomposition of urea are described below in examples.

The characteristics of the particles and the results of their research, as well as the content of the nitrogen fraction in the soil samples, are illustrated by the graphs, included in the figures:
- FIG. 1 -: graph - particle characterisation, in particular size and distribution, using the spectrometric method and the NANOPHOX instrument, for non-ionic silver particles with the trade name aXonnite^{®};
- FIG. 2 -: graph - content of mineral nitrogen fraction in soil samples - oilseed rape, BR_P - without plants start, BR_K - without plants end;
- FIG. 3 -: graph - mineral nitrogen fraction content of soil samples - maize, BR_P - without plants start, BR_K - without plants end;
- FIG. 4 -: graph - content of mineral nitrogen fraction in soil samples - wheat BR_P - without plants start, BR_K - without plants end;
- FIG. 5 -: graph - mineral nitrogen fraction content of soil samples - barley; BR_P - without plants start, BR_K - without plants end;
- FIG. 6 -: graph - results of factorial analysis of variance for ammonium nitrogen K - soil without fertiliser supplementation; M - soil with urea supplementation; M1 - soil with urea and non-ionic silver particles - 1ppm; M5 - soil with urea and non-ionic silver particles - 5 ppm; M10 - soil with urea and non-ionic silver particles - 10 ppm; M50 - soil with urea and non-ionic silver particles - 50 ppm;
- FIG. 7 -: graph - results of factorial analysis of variance for nitrite nitrogen K - soil without fertiliser supplementation; M - soil with urea supplementation; M1 - soil with urea supplementation and non-ionic silver particles - 1ppm; M5 - soil with urea supplementation and non-ionic silver particles - 5 ppm; M10 - soil with urea supplementation and non-ionic silver particles - 10 ppm; M50 - soil with urea supplementation and non-ionic silver particles - 50 ppm
- FIG. 8 -: graph 8 shows the concentration of ammoniacal nitrogen after urea decomposition catalysed by urease

Wherever non-ionic silver particles are referred to in the text, this refers to non-ionic silver particles obtained by a physical method, without impurities and with a purity of more than 99.99 %, in the form of particles with a developed active surface area, different sizes and a number distribution of these sizes from 1 nm to 500 nm.

Any physical method may be used to obtain such a form of silver, preferably the Bredig method of grinding the metal in an electric arc. The obtained non-ionic form of silver colloid does not require stabilisation, unlike the ionic form.

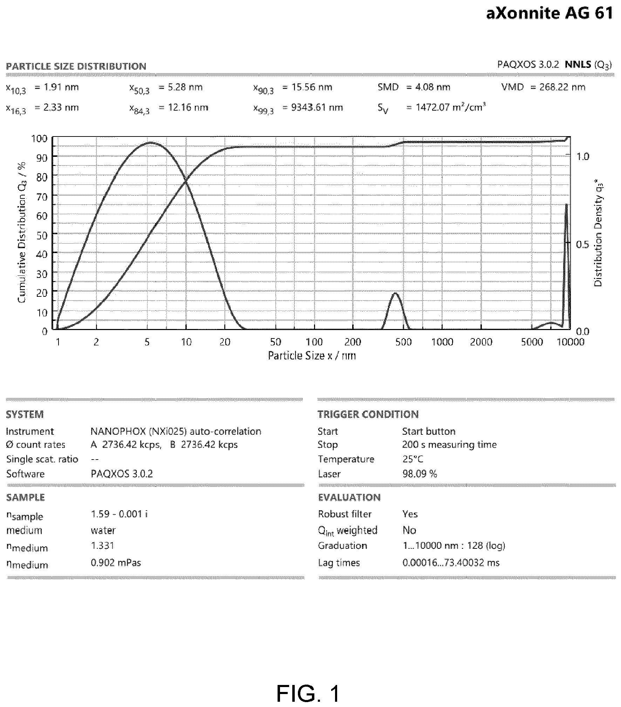

The characteristics of these particles, in particular their size and distribution, are shown in the diagram - fig. 1, using the spectrometric method and the NANOPHOX instrument.

This is a DLS detector, a particle size measurement device. Dynamic Light Scattering (DLS) is a method for measuring the size of nanoparticles dispersed in a liquid. In this method, the dispersion of nanoparticles is illuminated by a laser beam. The laser light is scattered and collected by a detector. The study concerns non-ionic silver particles with the trade name aXonnite^{®}.

### Example 1 - preparation of fertiliser

To prepare 100 g of fertiliser with a concentration of 1 ppm, 1 mL of a solution of nonionic silver particles with a concentration of 100 mg/L and 100 g of granular urea (analitically pure) were used. The fertiliser was produced using a diffusion method with penetration up to 50% (determined in the cross-section of urea grains). To prepare the fertiliser, granular urea was placed into a two-neck round-bottom flask with a ground joint, then the flask with the weighed sample was mounted on a Buchi ROTAVAPOR R-215 rotary evaporator. The device was then started, rotating the vessel at a speed of 5-15 rpm. Next, a nebuliser was used to apply the solution of silver particles onto the rotating granules, with an air flow rate of 2 ml/min. This procedure was repeated four times. Afterward, the vessel with its contents was dried at a temperature of 20-25°C with an air stream at a flow rate of 2.5 L/min until the granules achieved a water content of approximately 99.9%.

### Example 2 - preparation of fertiliser

To prepare 100 g of fertiliser with a concentration of 50 ppm, 50 mL of a solution of nonionic silver particles with a concentration of 100 mg/L and 100 g of granular urea (analitically pure) were used. The fertiliser was produced using a diffusion method with penetration up to 50% (determined in the cross-section of urea grains). To prepare the fertiliser, granular urea was placed into a two-neck round-bottom flask with a ground joint, then the flask with the weighed sample was mounted on a Buchi ROTAVAPOR R-215 rotary evaporator. The device was then started, rotating the vessel at a speed of 5-15 rpm. Next, a nebuliser was used to apply the solution of silver particles onto the rotating granules, with an air flow rate of 2 ml/min. This procedure was repeated four times. Afterward, the vessel with its contents was dried at a temperature of 20-25°C with an air stream at a flow rate of 2.5 L/min until the granules achieved a water content of approximately 99.9%.

The fertiliser produced in this way was applied to plants according to the examples below.

### Example 3 - fertiliser application to plants

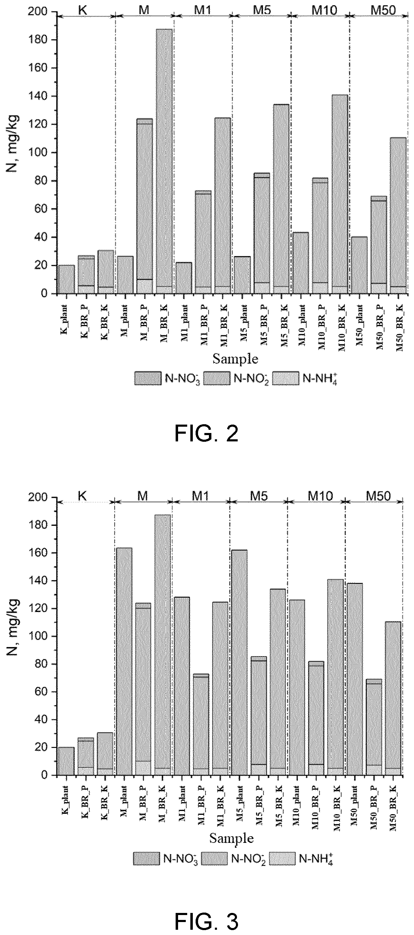

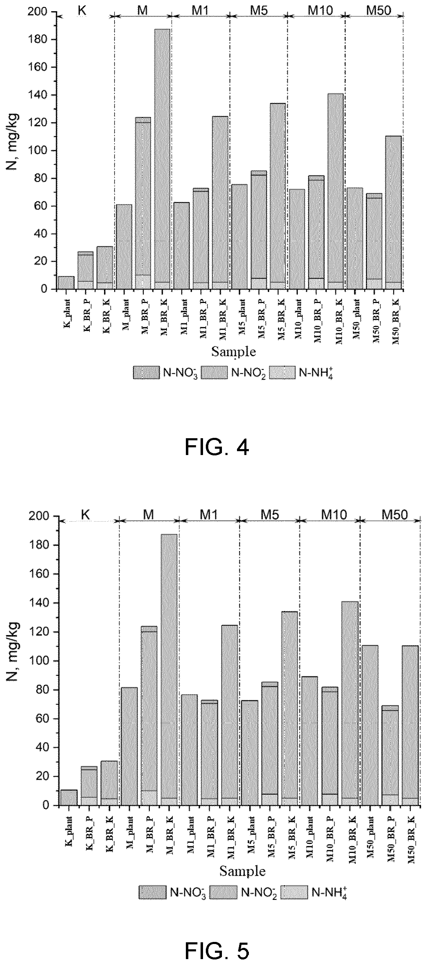

The content of mineral nitrogen fractions in soil samples, depending on the plant species, is presented in the graphs, fig. 2-5. These graphs also show the concentration of individual mineral nitrogen fractions for samples without plants at the beginning and end of the pot experiment. For the control sample without plants, fertilisation with the tested fertiliser compared to urea resulted in lower values of total inorganic nitrogen at the end of the experiment than for all tested fertilisers with the addition of nonionic silver particles. A similar trend was noted for corn, as shown in fig. 3. For other plants, the fertiliser with the addition of nonionic silver particles at a level of 50 ppm generally recorded the highest total mineral nitrogen content, as shown in fig. 2, 4, 5, which, as noted at the beginning of the paragraph, was still lower than for the sample without plants at the end of the experiment.

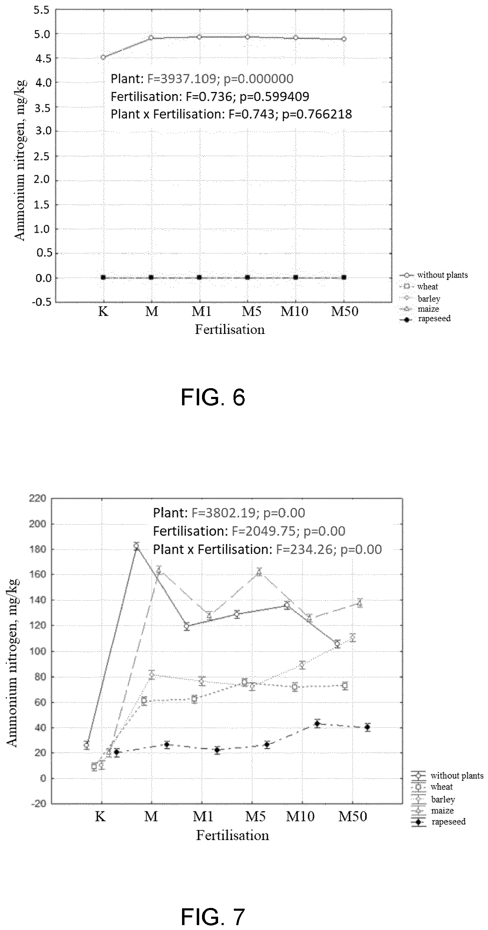

The concentrations of individual mineral nitrogen fractions were subjected to statistical analysis. The ammonium nitrogen content for samples with plants at the end of the experiment ranged from 0.002-0.006 mg/kg. These values, compared to urea alone, were higher for all additions of the tested fertiliser except for the M50 dose. The ammonium nitrogen content for samples without plants at the end of the experiment ranged from 4.52 to 4.93 mg/kg. High values of ammonium nitrogen were recorded in the sample without plants. For the M1 and M5 fertilisers, the ammonium nitrogen content is higher than for urea alone, indicating a slowing down of the process of converting organic nitrogen to ammonium nitrogen with the applied fertilisation. For the M10 and M50 samples, these results are slightly lower compared to the control sample with urea alone. Table 1 summarizes the ammonium nitrogen concentration in soils after the experiment. A factorial analysis of variance showed that only the type of plant had a statistically significant effect on the value of the discussed indicator, graph in fig. 6, as confirmed by a one-way analysis of variance conducted for all combinations (table 2).

**Table 1. Ammonium nitrogen (N-NH₄+) content in soils**

| | **Plant** | | | | | | | | | |
|---|---|---|---|---|---|---|---|---|---|---|
| | Rapeseed | | Maize | | Barley | | Wheat | | Soil without plants | |
| | average | SD | average | SD | average | SD | average | SD | average | SD |
| **K** | 0.0047 | 0.0002 | 0.0047 | 0.0002 | 0.0051 | 0.0001 | 0.0049 | 0.0001 | 4.5172 | 0.3976 |
| **M** | 0.0019 | 0.0002 | 0.0041 | 0.0000 | 0.0046 | 0.0004 | 0.0059 | 0.0002 | 4.9108 | 0.3711 |
| **M1** | 0.0033 | 0.0000 | 0.0048 | 0.0000 | 0.0052 | 0.0002 | 0.0052 | 0.0002 | 4.9295 | 0.1856 |
| **M5** | 0.0031 | 0.0001 | 0.0052 | 0.0002 | 0.0049 | 0.0000 | 0.0052 | 0.0001 | 4.9295 | 0.2916 |
| **M10** | 0.0031 | 0.0002 | 0.0050 | 0.0003 | 0.0049 | 0.0001 | 0.0055 | 0.0000 | 4.9108 | 0.1590 |
| **M50** | 0.0017 | 0.0001 | 0.0042 | 0.0002 | 0.0045 | 0.0002 | 0.0055 | 0.0001 | 4.8920 | 0.4506 |

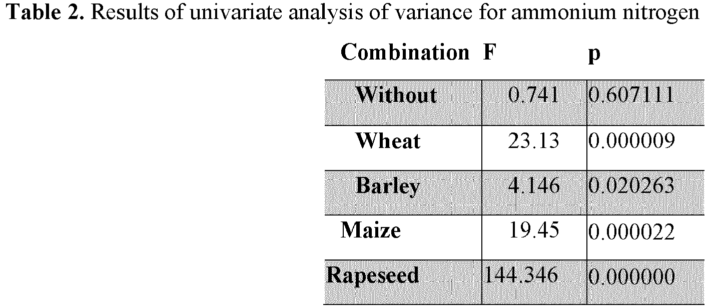

The content of nitrite nitrogen for samples without plants at the end of the experiment ranged from 0.05 to 0.08 mg/kg (Table 3). Similarly, as for nitrite nitrogen, a statistically significant effect on its value was noted for variables such as plant species, fertilisation method, and the interaction between them (graph fig. 7).

| **Table 3.** Content of nitrate nitrogen (N-NO₃) in soils | | | | | | | | | | |
|---|---|---|---|---|---|---|---|---|---|---|
| | **Plant** | | | | | | | | | |
| | Rapeseed | | Maize | | Barley | | Wheat | | Soil without plants | |
| | average | SD | average | SD | average | SD | average | SD | average | SD |
| **K** | 20.00 | 2.12 | 20.00 | 1.41 | 10.50 | 0.71 | 9.00 | 1.41 | 26.00 | 1.41 |
| **M** | 26.40 | 0.57 | 163.50 | 3.54 | 81.50 | 2.12 | 61.00 | 1.41 | 182.50 | 4.95 |
| **M1** | 21.95 | 2.19 | 128.00 | 4.24 | 76.50 | 2.12 | 62.50 | 2.12 | 119.50 | 2.12 |
| **M5** | 26.15 | 4.74 | 162.00 | 1.41 | 72.50 | 3.54 | 75.50 | 2.12 | 129.00 | 2.83 |
| **M10** | 43.20 | 2.55 | 126.00 | 2.83 | 89.00 | 1.41 | 72.00 | 2.83 | 136.00 | 4.24 |
| **M50** | 40.00 | 1.41 | 138.00 | 1.41 | 110.50 | 3.54 | 73.00 | 2.83 | 105.50 | 3.54 |

For all tested combinations, the univariate analysis of variance showed statistically significant differences between the samples (Tables 4-9). For the combinations without plants (Table 5) and for corn (Table 8), the highest concentration of nitrate nitrogen was observed in samples fertilised with urea alone. In contrast, for barley (Table 7) and rapeseed (Table 9), higher values of nitrate nitrogen compared to urea were noted for fertilisers with the addition of nonionic silver particles above 5 ppm. For wheat (Table 6), these values were observed for fertilisers with the addition of nonionic silver particles above 1 ppm, although no statistically significant differences were noted for M5, M10, and M50.

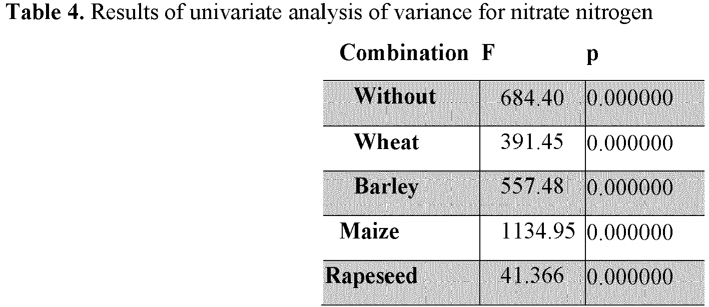

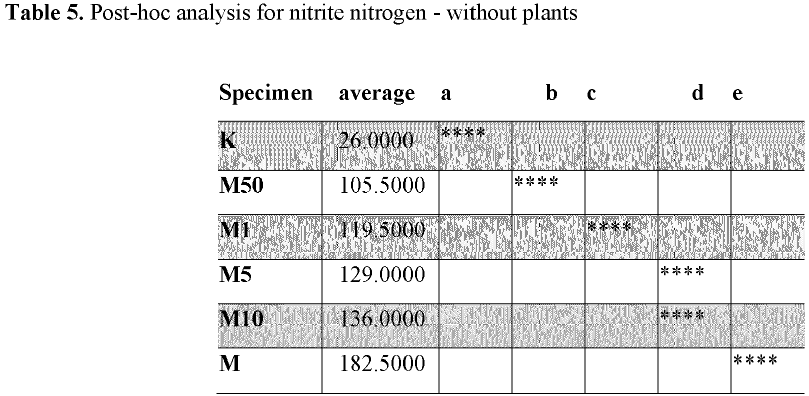

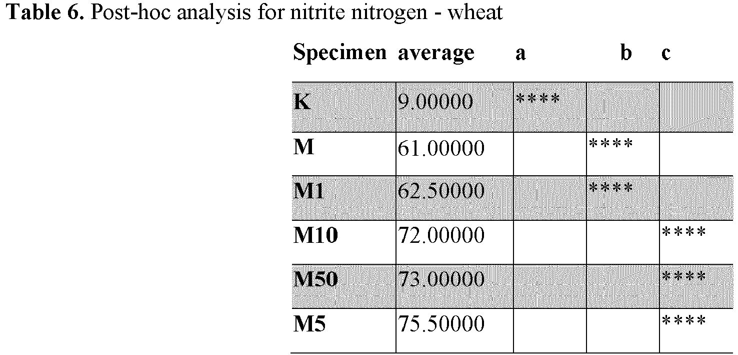

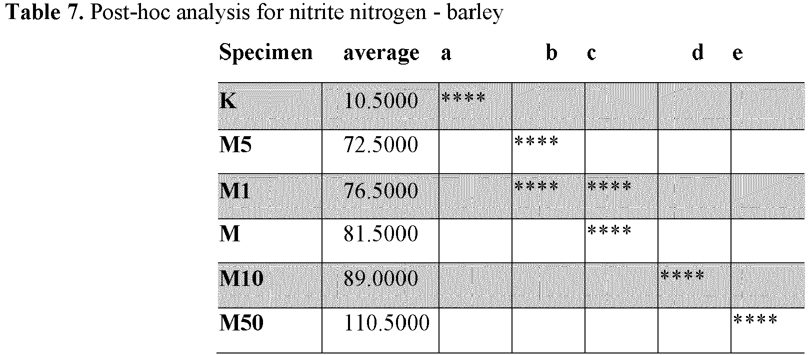

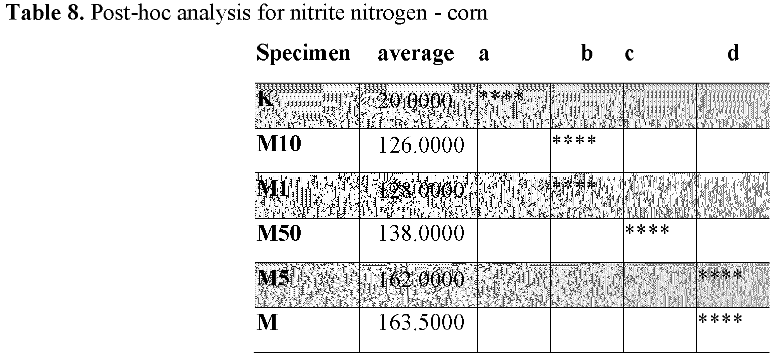

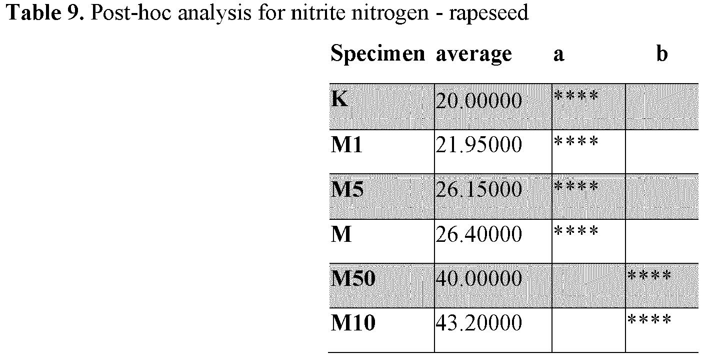

### Example 4 - study of urea decomposition

The effect of nonionic silver particles on urea decomposition catalysed by urease was studied by measuring changes in ammonium nitrogen concentration. This was done in model urea solutions (0.72 M) to which commercially available enzyme was added Optimal conditions for the enzymatic reaction were ensured by the addition of EDTA and phosphate buffer. After a 45-minute incubation, the enzymatic reaction was stopped, and the ammonium nitrogen concentration was determined spectrophotometrically using the phenol-hypochlorite method.

In this method, ammonia produced during the hydrolysis of urea reacts with sodium hypochlorite, forming chloramine, which in the presence of oxygen converts into p-chloroquinone imine. This compound then oxidizes with phenol to form a blue indophenol dye (formula below). The intensity of the blue colour is proportional to the ammonia content in the sample.

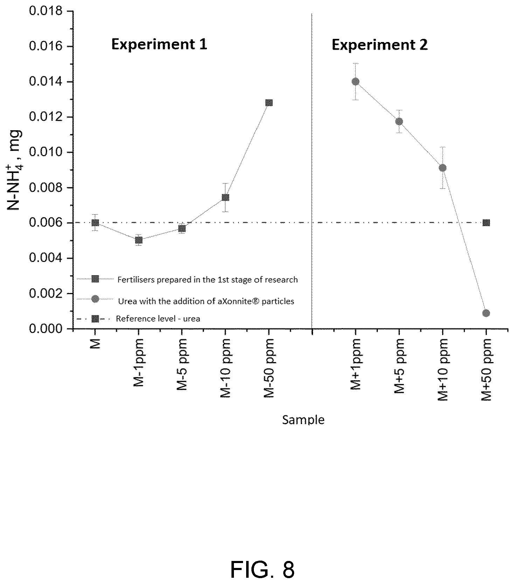

Based on the results of two experiments, the impact of nonionic silver particles on urea decomposition catalysed by urease was evaluated (graph Fig. 8). In the first experiment, model urea solutions were prepared from fertilisers obtained in the first stage of the research. In the second experiment, the appropriate concentration of nonionic silver particles was added to the urea to achieve a concentration in the model urea solution analogous to that in the compatible fertiliser.

In the first experiment, inhibition of urease was observed at nonionic silver particle concentrations of 1 ppm and 5 ppm. Compared to urea alone, the ammonium nitrogen concentration for the fertiliser with the addition of 1 ppm nonionic silver particles was 16% lower.

The reaction scheme is presented below:

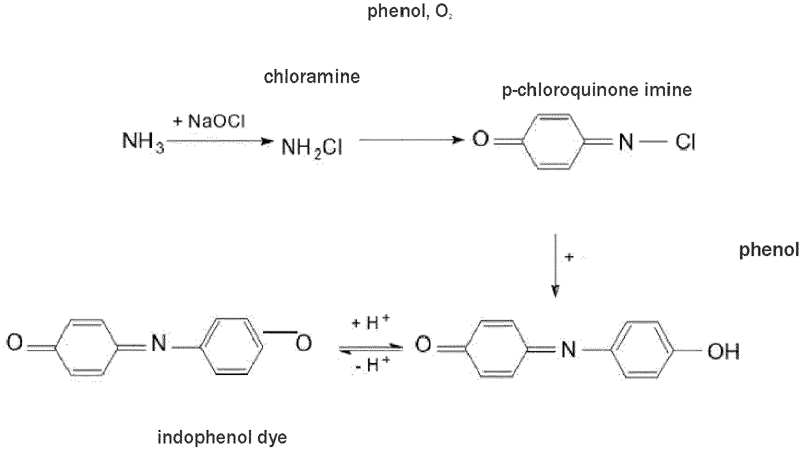

In the second experiment, the ammonium nitrogen concentration decreased with increasing addition of nonionic silver particles to the model urea solution. However, urease inhibition was only noted for the sample with 50 ppm of nonionic silver particles. In other cases, after the enzymatic reaction was completed, the ammonium nitrogen concentration was higher than for urea alone.

## Claims

1. Method for slowing down the decomposition of urea in the soil contained in nitrogen fertilisers used for affecting plants, by applying a urease inhibitor - an enzyme that catalyses the decomposition of urea in the fertiliser to ammonia and carbon dioxide during the hydrolysis process occurring in the soil, **characterised in that** the urease inhibitor used is non-ionic silver particles, obtained by a physical method, without impurities and with a purity above 99.99%, in the form of particles with an expanded active surface area, various sizes, and a number distribution of these sizes from 1 nm to 500 nm, wherein the silver particles are dosed into the fertiliser mass in the form of a colloid containing the mentioned silver particles in deionized water, to achieve a concentration in the fertiliser mass from 1 to 50 ppm.

2. Method according to claim 1, **characterised in that** the interaction with the nitrogen fertiliser is carried out on plants sown in the spring.

3. Method according to claim 1, **characterised in that** the interaction with the nitrogen fertiliser is carried out on cereal plants, including wheat, corn, and rapeseed.

4. Nitrogen fertiliser with a urease inhibitor that slows down the decomposition of the urea contained in the fertiliser, **characterised in that** the granulated fertiliser contains non-ionic silver particles as the urease inhibitor, obtained by a physical method, with a purity above 99.99%, in the form of particles with an expanded active surface area and a number size distribution from 1 nm to 500 nm, initially introduced in the form of a colloid containing the mentioned silver particles in deionized water, with a concentration in the fertiliser mass from 1 ppm to 50 ppm.

5. Use of non-ionic silver particles, obtained by a physical method, without impurities and with a purity above 99.99%, in the form of particles with an expanded active surface area, various sizes, and a number distribution of these sizes from 1 nm to 500 nm, introduced into the fertiliser in the form of a colloid containing the mentioned silver particles in deionized water, with a concentration in the fertiliser mass from 1 ppm to 50 ppm, as a urease inhibitor that slows the decomposition of urea contained in the nitrogen fertiliser - to slow down and extend the gradual uptake of nitrogen by plants.
